# EUROPEAN PATENT SPECIFICATION

(11) **EP 3 110 839 B1**
(45) Date of publication and mention of the grant of the patent: **16.05.2018**
(21) Application number: 15710723.6
(22) Date of filing: 23.02.2015
(51) Int. Cl.: C07K 14/59

(54) **EXTRACTION PROCESS TO OBTAIN HCG HAVING A HIGH BIOLOGICAL ACTIVITY**
EXTRAKTIONSVERFAHREN ZUR GEWINNUNG VON HCG MIT HOHER BIOAKTIVITÄT
PROCÉDÉ D'EXTRACTION POUR OBTENIR DE LA HCG POSSÉDANT UNE ACTIVITÉ BIOLOGIQUE ÉLEVÉE

(30) Priority: 24.02.2014 IT MI20140274
(43) Date of publication of application: 04.01.2017
(73) Proprietor: Altergon S.A., 6900 Lugano (CH)
(72) Inventor: SECONDI, Roberto, CH-6900 Lugano (CH); CACCIA, Paolo, CH-6900 Lugano (CH)
(74) Representative: Gerli, Paolo
(86) International application number: PCT/EP2015/053691
(87) International publication number: WO 2015/124759

(56) References cited:
- WO-A1-2010/145125
- WO-A2-2007/065918
- CN-A- 101 792 481
- I. FELLEGVÁRI ET AL: "Purification of human chorionic gonadotropin hormone by anion-exchange high-performance liquid chromatography", CHROMATOGRAPHIA, vol. 27, no. 11-12, 1 June 1989 (1989-06-01), pages 601-604, XP055145430, ISSN: 0009-5893, DOI: 10.1007/BF02258986
- PRASAD PRAMOD V ET AL: "Isolation of hCG and its characterization by radioimmunoassay, enzyme-immunoassay, and radio-receptor assay", JOURNAL OF IMMUNOASSAY AND IMMUNOCHEMISTRY, DEKKER, NEW YORK, NY, US, vol. 26, no. 4, 1 January 2005 (2005-01-01), pages 325-344, XP008172653, ISSN: 1532-1819, DOI: 10.1080/15321810500220951 [retrieved on 2007-02-06]
- MAHMOOD H. QAZI ET AL: "Preparation of Highly Purified Human Chorionic Gonadotrophin by Isoelectric Focusing", EUROPEAN JOURNAL OF BIOCHEMISTRY, vol. 47, no. 2, 1 September 1974 (1974-09-01), pages 219-223, XP055146829, ISSN: 0014-2956, DOI: 10.1111/j.1432-1033.1974.tb03685.x

## Description

### PRIOR ART

Together with follicle-stimulating hormone (FSH) and luteinizing hormone (LH), chorionic gonadotropin forms the group of gonad-stimulating hormones, generally referred to as gonadotropins.

hCG is produced by the outer membrane of a fertilised egg and subsequently by the placenta. It serves the function of prolonging the effect of the luteinizing hormone on the corpus luteum during pregnancy; when suitably stimulated, the latter takes the consistency typical of the corpus luteum of pregnancy, and secretes high quantities of progesterone, a hormone that is necessary for the development of pregnancy.

The hCG molecule is a heterodimer consisting of two subunits (α and β). Subunit α has a structure identical to that of the other gonadotropins (LH and FSH), whereas subunit β is specific for each hormone. hCG may be dosed in biological liquids with immunometric methods (radioimmunological method, immunoenzymatic method, chemiluminescence). The presence of hCG in urine implies that the embryo has implanted itself: for this reason it is used as a diagnostic indicator in the most common pregnancy tests.

In the pharmaceutical industry, hCG is used to stimulate ovulation and to treat female infertility. It has effects on men, too, as it stimulates testicular development.

Chromatographic methods for purifying hGC are disclosed in CN101792481A, Fellegvari et al (Chromatographia, 27(11-12), 1989, 601-4), Prasad et al (J Immunoassay Immunochem, 26(4), 2005, 325-44) or Qazi et al (J Biochem, 47(2), 1974, 219-23).

The quality of the hCG preparations for pharmaceutical use is characterised on the basis of the specific biological activity that measures the real potency of the product; this value does not depend only on the absolute quantity of hCG in the preparation, but also on the composition of hCG in its various isoforms. Indeed hCG is a composite product, i.e., a mixture of isoforms having different degrees of activity: a preparation may contain more hCG than another one, and yet have a lower biological activity if it is rich in inactive isoforms; vice versa, a preparation may contain less hCG than another one, and yet have a higher biological activity if it is richer in active isoforms.

The hCG available on the market either may be obtained by extraction from gravidic urine or is produced by recombination. The recombinant technique provides a product having reproducible characteristics of a medium-high activity, and free from contaminants (allergens), but features high production costs. Hence the interest, still high today, in maintaining/improving the extraction methods.

The extraction methods are substantially based on a first phase of purification of crude urine, in order to remove any non-protein, low-molecular-weight components, and reduce any bacterial and possibly viral load present in urine. This phase involves important purification operations (several selective extractions and precipitations, whereby the material is contacted with different levels of alcohol, filter precipitated, resuspended, etc.). At the end of this phase there is obtained a hCG fraction of an intermediate degree of purity and having still very low specific activity. The second phase is based on chromatographic separations with elution and recovery of specific hCG-enriched fractions. However, normally the specific activity of the hCG thus obtained does not turn out to be high - though it meets the minimum requirements for pharmaceutical use - as the product may undergo a partial degradation during the processing phases. Moreover, sometimes the quantitative process yield is low, since the extraction of hCG from the starting crude material is not always optimum.

By causing a partial degradation of the glycoprotein structure, the current extraction processes increase the percentage of poorly active or inactive isoforms. Therefore, in order to maintain a sufficiently high process yield it is necessary to recover most of the hCG, thereby including poorly active fractions and thus lowering the specific activity of the final product; vice versa, should one collect only hCG with sufficiently high activity, then one is forced to reject all poorly active/inactive hCG fractions, thus strongly reducing the process yield.

Thus, there is a strong demand for novel processes for the extraction - production of hCG that lead to a pharmaceutical grade product having a high specific biological activity, allowing at the same time a high process yield.

### SUMMARY

The Applicant has now developed a novel process for the extraction of hCG from urine - highly respectful of the native hCG - by means of which there is obtained pharmaceutical grade hCG in high quantitative yield, while avoiding or strongly reducing at the same time the degradation that occurs with known extraction processes.

The inventive process is characterised by the following phases:
(a) separation of urine of pregnant women, or crude fraction thereof, by chromatography on ion exchange resin having a particle size between 50 and 500 micrometers;
(b) separation of the product obtained in (a), by means of dye-affinity chromatography;
(c) ultra/nanofiotration of the solution obtained in (b);
(d) recovery of the final product from the solution obtained in (c).

This novel process allows to work directly on native urine, such that the preliminary phase of purification of crude urine prior to chromatography becomes optional; should this be optionally implemented in the present process, too, (e.g., in the case of production facilities not designed for the treatment of high volumes of urine), it may have a minor impact, i.e., be significantly "milder" (shorter, simpler) than the procedures commonly used; in particular, it may occur by means of a single step of precipitation of urine protein material, the said precipitation being typically obtained with benzoic acid, or salt thereof. Indeed, thanks to the high discriminating power of the chromatographic steps implemented in the invention, a poor or absent initial purification of the starting urine does not really affect the quantitative process yield; rather, this circumstance contributes to protect the natural active isoforms of hCG, thus avoiding or reducing its degradation in inactive isoforms, and thus allowing the recovery of hCG having a high specific activity (greater than 12000 IU/mg, corresponding to the best hCG preparations currently available on the market), in a high quantitative yield. The elimination of the lengthy pre-chromatographic processes of urine purification, currently required in the prior art, also contributes to the simplicity and economy of the process.

### DETAILED DESCRIPTION

### Starting material

The starting material for the present process may be urine of pregnant women, or crude fraction thereof.

The urine of pregnant women may be used as it is, except for a possible preliminary clarification by filtering the material in suspension and by adjusting the pHs of the urine batches within a standard operating range, typically between 4 and 6.

Alternatively, as a starting material the process may use a crude fraction of the urine itself. Advantageously, in the present process such crude fraction may be obtained by means of a single precipitation of the protein component, whereby preferably the latter may be obtained by treating urine with a suitable organic acid, or salt thereof, typically benzoic acid or sodium benzoate; typically, precipitation is carried out at a temperature comprised between 4 and 12°C, e.g., at 8±2°C, at a pH comprised between 3 and 5, e.g., between 3.6 and 4.0; the protein precipitate is recovered, dried and washed with suitable solvents, e.g., ethanol, acetone, ethyl ether. The crude fraction thus obtained exhibits a low hCG-specific biological activity, generally comprised between 1 and 10 IU/mg.

In preparation for phase (a) of the present process, the crude hCG fraction is extracted in a suitable solvent, preferably a hydroalcoholic solution having a pH between 4 and 6, and after separation of the insoluble particulate matter through centrifugation, the supernatant is applied on the chromatographic column used in phase (a).

### Phase a)

This first chromatographic separation is carried out on an ion exchange resin having a particle size comprised between 50 and 500 microns, preferably between 100 and 300 microns; the resin is preferably crosslinked, and contains agarose; furthermore, it has binding capacity comprised between 0.05 and 0.50 mmol of protein/ml of resin. A particularly preferred resin is SP Sepharose Big Beads, GE Healthcare.

In phase (a), the starting urine, or crude fraction thereof as specified above, is applied to the column. After a preliminary washing, the column is eluted with a hydroalcoholic solution having a pH comprised between 4 and 6, preferably between 4.8 and 5.4; the said pH is determined by the presence of a suitable salt in solution, typically ammonium acetate. The eluate is collected in fractions by recovering those containing hCG. The amount of hCG present in the fractions may be detected by measuring the immunoenzymatic titer.

The pool of recovered fractions then undergoes a standard diafiltration post-treatment, followed by precipitation with ethanol.

If the starting material used was a crude urine fraction, at this stage the product has a hCG-specific activity generally in excess of 4000 IU/mg, with a purification factor (meant as the ratio between the specific activity of the product before and after the chromatographic step under consideration) higher than 400. By contrast, if the starting material was native urine, here the product has a hCG-specific activity generally not lower than 500 IU/mg, with a purification factor not lower than 3000.

### Phase (b)

The precipitate obtained at the end of phase (a) is dissolved in a suitable solvent, preferably an aqueous solution having a pH between 4 and 6, and applied to the column.

The column used in phase (b) uses a dye-affinity resin, preferably containing agarose; a particularly preferred resin is Blue Sepharose 6 Fast Flow.

After a preliminary washing, the column is eluted with a linear gradient of both salt concentration and pH; the gradient of salt concentration (e.g., obtained with KCl) preferably varies between 0.1 and 3.0 M, more preferably between 0.3 and 2.2 M KCl; and the pH varies between 11 and 8, more preferably between 10 and 9. The eluate is collected in fractions, by recovering those containing hCG, which may be detected by determining the immunoenzymatic titer.

### Phase (b1)

The production process may optionally comprise an additional chromatographic separation, too, carried out on an ion exchange resin. In this case, a preferred resin is DEAE Sepharose Fast Flow. To this end, on the column there is applied the solution containing hCG, resulting from phase (b). After a preliminary washing, the column is eluted with a hydroalcoholic solution having a pH of about 7, with a linear gradient of salt concentration; the gradient - generally obtained with sodium phosphate - varies between 10 and 100 mM, more preferably between 25 and 75 mM. The eluate is collected in fractions, by recovering those containing hCG, which may be determined by measuring the immunoenzymatic titer.

### Phase (c)

This phase involves a set of ultrafiltration steps - necessary to concentrate the solutions and remove low-molecular-weight components - and nanofiltration steps - to eliminate any viruses possibly present in solution. Preferably there are carried out a nanofiltration step and at least two ultrafiltration steps. The nano/ultrafiltration steps may be carried out in any order; preferably, nanofiltration is intermediate between two ultrafiltration steps.

### Phase (d)

Phase (d) involves final purification and recovery of hCG. It involves a step of selective precipitation of hCG from the related aqueous phase resulting from (c), obtained by means of suitable alcohols, base and salts, at a pH comprised between 8 and 10; the preferred alcohols, base and salts for this selective precipitation are ethanol, ammonium hydroxide, ammonium acetate, calcium acetate and tribasic dodecahydrate sodium phosphate, respectively.

The precipitate thus obtained is washed with alcohol, preferably ethanol, and recovered through centrifugation; the supernatant may be further treated to recover any traces of hCG left in solution.

If a crude urine fraction was used as a starting material, the final product thus obtained has a hCG-specific biological activity generally in excess of 15000 IU/mg, with a purification factor related to this process phase greater than 3.5.

By contrast, if the starting material was native urine, the product thus obtained has a hCG-specific biological activity generally in excess of 12000 IU/mg, with a purification factor greater than 20.

One may note that the final hCG-specific activity is high and has the same order of magnitude (12000-15000 IU/mg), though the starting material is native urine or a crude fraction thereof, both being highly impure; this may be attributed to the high discrimination capacity of the chromatographic system used, capable of very effectively separating all hCG present, in a way that is strongly independent of the degree of purity of the starting urine material.

The inventive process is illustrated hereinbelow through the following, non-limiting examples.

### EXAMPLE 1: Process carried out on a crude urine fraction

### Preparation of fraction "A" (crude hCG 5-10 IU/mg) and extraction thereof

Urine is received in the production facilities and transferred to the process tanks. There is added 2% (w/v) of sodium benzoate, and the pH is adjusted between 3.6 and 4.0 by adding 20% HCl under stirring. The protein precipitate thus obtained is recovered through filtration.

The precipitate is then washed with suitable solvents and vacuum dried.

### (Fraction A)

In order to bring hCG into a liquid phase applicable to chromatography, 100 kg of Fraction "A" are poured - under continuous stirring - into 1000 litres of a solution - previously cooled down (5°C) - of 0.1M ammonium acetate at a pH of 5.0 - 5.2 with 10% ethanol, and the resulting suspension is stirred for 3 hours. The suspension is then centrifuged for 30 minutes at > 4000 rpm. The supernatant containing hCG is separated and collected.

### Phase (a) Chromatography on SP Sepharose Big Beads

Resin: SP Sepharose Big Beads, GE Healthcare;
Composition : highly cross-linked 6 % agarose / Sulphopropyl
Particle size : 100-300 µm (micrometers).
Ion capacity (binding capacity): 0.18-0.25 mmol / ml medium
pH stability (working) 4-13
Cip stability (short-term) 3-14
Spec. pressure/flow rate 1200-1800 cm/h.
Equilibration buffer: 0.1 M ammonium acetate at a pH of 5.0 -5.2, 10 % EtOH.

The supernatant obtained after centrifugation is applied on the SP Sepharose Big Beads column at a flow rate of 80 L/h. The column is then washed with the following solutions:
No. 1) 0.1 M ammonium acetate, pH: 5.0-5.2, 40% (v/v) ethanol, flow rate: 80 L / h.
No. 2) 0.3 M ammonium acetate, pH: 5.0-5.2, 40% (v/v) ethanol, flow rate: 80 L / h.

The column is then eluted with a solution of 0.7 M ammonium acetate, pH: 5.0-5.2, with 40% (v/v) ethanol, flow rate : 60 L/h.

The eluate is collected in fractions; the hCG content is evaluated by immunoenzymatic assay and the total protein concentration by spectrophotometric measurement.

Then, the fractions containing hCG are collected and mixed, and then concentrated and diafiltered through a cartridge having a cut-off of 10kDa, thereby obtaining a final protein solution in purified water. The hCG content is evaluated by immunoenzymatic assay and the total protein concentration by spectrophotometric measurement.

Then, four volumes of 96% ethanol previously cooled down are added to each volume of hCG solution, and left to stand for at least 3 hours at 5±3°C.

Subsequently, the precipitate is recovered through centrifugation, dissolved in purified water, and the amount of hCG is evaluated by immunoenzymatic assay. The total protein concentration is measured by spectrophotometric determination. The solution is stored at 5 ± 3 °C.

The specific activity of the solution turns out to be not lower than 4000 IU / mg, with a purification factor not lower than 400.

### Phase (b) Chromatography on Blue Sepharose 6 fast flow.

The solution is equilibrated in 20 mM sodium acetate, pH 5.5, and applied on a chromatographic column containing the Blue-Sepharose 6 Fast Flow resin.

The column is then washed with the following buffers:
Buffer 1) 50 mM glycine - NaOH, pH 10
Buffer 2) 0.2 M KCl in 50 mM glycine - NaOH, pH 9

Then, the column is eluted with a linear gradient from:
0.3 M KCl in 50 mM glycine - NaOH, pH 10 (20 L) to:
2.2 M KCl in 50 mM glycine - NaOH, pH 9 (20 L)

The eluate is collected in fractions. The hCG content is evaluated by immunoenzymatic assay and the total protein concentration by spectrophotometric measurement. The fractions containing hCG are then collected and gathered in a single pool.

### Phase (b1) Chromatography on DEAE Sepharose Fast Flow

Optionally, the pool of fractions resulting from the Blue Sepharose 6 Fast Flow chromatography is diafiltered and equilibrated in a buffer of 10 mM sodium phosphate, pH 7.3, 10 % (v/v) ethanol.

The solution thus obtained is applied on a chromatographic column containing the resin DEAE Sepharose Fast Flow. Then, the resin is washed with the following buffer: 22 mM sodium phosphate, pH 7.3, 10 % (v/v) ethanol (v/v) and the proteins eluted with a linear gradient from:
25 mM sodium phosphate, pH 7.3, 10 % (v/v) ethanol, 50 litres to:
75 mM sodium phosphate, pH 7.3 , 10 % (v/v) ethanol, 50 litres

Then, the eluate is collected in fractions.

The hCG content is evaluated by immunoenzymatic assay and the total protein concentration by spectrophotometric measurement. The fractions containing hCG are then collected and gathered in a single pool.

### Phase (c) ultrafiltration / nanofiltration

The pools coming from the fractions collected from the chromatographies described above (b and b1) are ultrafiltered on a 10 KDa membrane and equilibrated in a solution of 0.2 M KCl in 50 mM glycine, pH 7.5; then, the solution is filtered through a disposable, 0.22-micron filtering unit.

Subsequently, the resulting solution is nanofiltered by using a suitable system, at a constant pressure of 2 bar. The filtrate is collected and the solution is ultrafiltered to obtain a final protein solution in purified water. The hCG content is evaluated by immunoenzymatic assay and the total protein concentration by spectrophotometric measurement.

### Phase (d) Isolation by selective precipitation and recovery of final hCG

For each 100 ml of ultrafiltered solution there are added: 19.4 g of ammonium acetate, 83.3 ml of 96 % ethanol (previously cooled down at - 20°C), 4.4 ml of a 7.6% tribasic dodecahydrate sodium phosphate solution, 5.0 ml of a 4.9% calcium acetate solution, 12 ml of 5 M sodium hydroxide at pH 9.4. After stirring for one hour, the solution is centrifuged for 30 minutes at 7000 rpm, and the supernatant is collected.

Then, two volumes of 96% ethanol - previously cooled down - are added to each volume of the collected supernatant. The resulting suspension is left to stand for at least 12 hours at -20 ± 5°C and then centrifuged for 30 minutes at 7000 rpm.

The precipitate obtained is dissolved in water for injection (WFI) and four volumes of 96% ethanol are added for each volume of the solution in order to reprecipitate the proteins, leaving then the suspension to stand for two hours at -20 ± 5°C.

Then, the suspension is centrifuged as described above.

The precipitate obtained is redissolved in WFI, and consists of hCG with a specific biological activity > 15000 IU / mg and with a purification factor greater than 3.5.

### EXAMPLE 2: Process carried out on native urine

Urine is received in the production facilities and transferred to the process tanks, and ammonium acetate is added thereto up to a concentration of 0.1M. The pH of the urine is titrated between 4.6 and 5.2 with acetic acid, and the urine is filtered through a 10-micrometer filtering unit.

The solution obtained is applied on a column of SP Sepharose Big Beads, and from this point there is followed the same procedure as that of the phases (a)-(d) shown in Example 1.

The precipitate obtained at the end of phase (d), redissolved in WFI, consists of hCG with a specific biological activity > 12000 IU / mg and with a purification factor ≥ 20.

## Claims

1. Process for the preparation of pharmaceutical grade human chorionic gonadotropin (hCG) comprising the phases:
(a) separation of urine of pregnant women, or crude fraction thereof, by chromatography on ion exchange resin having a particle size between 50 and 500 micrometers;
(b) separation of the product obtained in (a), by means of dye-affinity chromatography.
(c) ultra/nanofiltration of the solution obtained in (b)
(d) recovery of the final product from the solution obtained in (c).

2. Process according to claim 1, wherein the resin used in phase (a) is crosslinked.

3. Process according to claims 1-2, wherein the resin used in phase (a) has binding capacity comprised between 0.05 and 0.50 mmol protein/ml resin.

4. Process according to claims 1-3, wherein in phase (a) a hydroalcoholic eluent is used having pH between 4 and 6.

5. Process according to claims 1-4, wherein the crude urine fraction is obtained via a single step of precipitating the protein material from said urine.

6. Process according to claim 5, wherein the precipitation is carried out by means of a suitable organic acid or salt thereof.

7. Process according to claims 1-6, wherein in phase (b) an eluent is used with a gradient of salt concentration and of pH.

8. Process according to claim 7, wherein said salt concentration gradient varies between 0.1 and 3.0 M and said pH gradient varies between pH 11 and 8.

9. Process according to claims 1-8, wherein the resins used in phases (a) and/or (b) contain agarose.

10. Process according to claims 1-9, in which phase (c) comprises at least two steps of ultrafiltration and one step of nanofiltration, which can be carried out in any order.

11. Process according to claims 1-10, wherein phase (b) further includes a step (b1) of chromatographic separation on an ion exchange resin.

12. Process according to claims 1-11, wherein phase (d) comprises a selective precipitation of hCG from said solution in the presence of suitable alcohol, base and salts, at a pH between 8 and 10, and wherein the precipitate thus obtained is optionally dissolved and reprecipitated.

## Patentansprüche

1. Verfahren zur Herstellung von humanem Choriongonadotropin (hCG) von pharmazeutischer Qualität, folgende Phasen aufweisend:
(a) Trennen von Urin schwangerer Frauen, oder einer Rohfraktion davon, durch Chromatographie auf Ionenaustauscherharz mit einer Partikelgröße zwischen 50 und 500 Mikrometern;
(b) Trennen des in (a) erhaltenen Produkts mittels Farbaffinitätschromatographie;
(c) Ultra-/Nanofiltration der in (b) erhaltenen Lösung;
(d) Gewinnen des Endprodukts aus der in (c) erhaltenen Lösung.

2. Verfahren nach Anspruch 1, wobei das in Phase (a) verwendete Harz vernetzt ist.

3. Verfahren nach den Ansprüchen 1 bis 2, wobei das in Phase (a) verwendete Harz eine Bindungskapazität zwischen 0,05 und 0,50 mmol Protein/ml Harz hat.

4. Verfahren nach den Ansprüchen 1 bis 3, wobei in Phase (a) ein wässrigalkoholisches Elutionsmittel mit einem pH zwischen 4 und 6 verwendet wird.

5. Verfahren nach den Ansprüchen 1 bis 4, wobei die Rohurinfraktion mittels eines einzigen Schritts des Ausfällens des Proteinmaterials aus dem Urin erhalten wird.

6. Verfahren nach Anspruch 5, wobei das Ausfällen mittels einer geeigneten organischen Säure oder eines Salzes davon durchgeführt wird.

7. Verfahren nach den Ansprüchen 1 bis 6, wobei in Phase (b) ein Elutionsmittel mit einem Gradienten der Salzkonzentration und des pH verwendet wird.

8. Verfahren nach Anspruch 7, wobei der Salzkonzentrations-Gradient zwischen 0,1 und 3,0 M variiert und der pH-Gradient zwischen pH 11 und 8 variiert.

9. Verfahren nach den Ansprüchen 1 bis 8, wobei die in den Phasen (a) und/oder (b) verwendeten Harze Agarose enthalten.

10. Verfahren nach den Ansprüchen 1 bis 9, bei dem Phase (c) mindestens zwei Ultrafiltrationsschritte und einen Nanofiltrationsschritt aufweist, die in einer beliebigen Reihenfolge durchgeführt werden können.

11. Verfahren nach den Ansprüchen 1 bis 10, wobei Phase (b) außerdem einen Schritt (b1) der chromatographischen Trennung auf einem Ionenaustauscherharz umfasst.

12. Verfahren nach den Ansprüchen 1 bis 11, wobei Phase (d) eine selektive Ausfällung von hCG aus der Lösung in Anwesenheit von geeignetem Alkohol, Base und Salzen, bei einem pH zwischen 8 und 10 aufweist, und wobei die so erhaltene Ausfällung optional gelöst und erneut ausgefällt wird.

## Revendications

1. Procédé de préparation de la gonadotrophine chorionique humaine (hCG) de qualité pharmaceutique comprenant les phases suivantes :
(a) séparation de l'urine de femmes enceintes, ou d'une fraction brute de celle-ci, par chromatographie sur une résine échangeuse d'ions ayant une taille de particules comprise entre 50 et 500 micromètres;
(b) séparation du produit obtenu en (a), au moyen d'une chromatographie d'affinité de colorant,
(c) ultra / nanofiltration de la solution obtenue en (b)
(d) récupération du produit final à partir de la solution obtenue en (c).

2. Procédé selon la revendication 1, dans lequel la résine utilisée dans la phase (a) est réticulée.

3. Procédé selon les revendications 1 et 2, dans lequel la résine utilisée dans la phase (a) a une capacité de liaison comprise entre 0,05 et 0,50 mmol de protéine / ml de résine.

4. Procédé selon les revendications 1 à 3, dans lequel, dans la phase (a), on utilise un éluant hydroalcoolique ayant un pH compris entre 4 et 6.

5. Procédé selon les revendications 1 à 4, dans lequel la fraction d'urine brute est obtenue par une seule étape de précipitation de la matière protéique à partir de ladite urine.

6. Procédé selon la revendication 5, dans lequel la précipitation est réalisée au moyen d'un acide organique approprié ou d'un sel de celui-ci.

7. Procédé selon les revendications 1 à 6, dans lequel, dans la phase (b), on utilise un éluant avec un gradient de concentration en sel et de pH.

8. Procédé selon la revendication 7, dans lequel ledit gradient de concentration en sel varie entre 0,1 et 3,0 M et ledit gradient de pH varie entre un pH de 11 et de 8.

9. Procédé selon les revendications 1 à 8, dans lequel les résines utilisées dans les phases (a) et / ou (b) contiennent de l'agarose.

10. Procédé selon les revendications 1 à 9, dans lequel la phase (c) comprend au moins deux étapes d'ultrafiltration et une étape de nanofiltration, qui peut être réalisée dans n'importe quel ordre.

11. Procédé selon les revendications 1 à 10, dans lequel la phase (b) comprend en outre une étape (b1) de séparation chromatographique sur une résine échangeuse d'ions.

12. Procédé selon les revendications 1 à 11, dans lequel la phase (d) comprend une précipitation sélective de hCG depuis ladite solution en présence d'alcool, de base et de sels appropriés, à un pH compris entre 8 et 10, et dans lequel le précipité ainsi obtenu est éventuellement dissous et reprécipité.
